# EUROPEAN PATENT APPLICATION

(11) **EP 3 427 765 A1**
(43) Date of publication of application: **16.01.2019**
(21) Application number: 17763589.3
(22) Date of filing: 09.03.2017
(51) Int. Cl.: A61L 27/36, C12N 5/077, A61L 27/50, A61L 27/52, A61L 27/56

(54) **METHOD FOR PRODUCING EXTRACELLULAR MATRIX MEMBRANE DERIVED FROM BIOCOMPATIBLE PORCINE CARTILAGE CAPABLE OF REGULATING IN VIVO DECOMPOSITION RATE AND PHYSICAL PROPERTIES, AND COMPOSITION FOR PREVENTING ADHESION CONTAINING EXTRACELLULAR MATRIX DERIVED FROM PORCINE CARTILAGE AS ACTIVE INGREDIENT**

(30) Priority: 11.03.2016 KR 20160029579
(71) Applicant: ATEMs Co., Ltd., Gyeonggi-do 16226 (KR)
(72) Inventor: MIN, Byoung-Hyun, Anyang-si Gyeonggi-do 14101 (KR); KIM, Young Jick, Gimhae-si Gyeongsangnam-do 50836 (KR); SONG, Bo Ram, Seoul 07362 (KR); YUN, Hee Woong, Seoul 03088 (KR); JEONG, Sung In, Chungju-si Chungcheongbuk-do 27417 (KR); LIM, Youn-Mook, Jeongeup-si Jeollabuk-do 56196 (KR); PARK, Jong-Seok, Iksan-si Jeollabuk-do 54661 (KR); GWON, Hui-Jeong, Jeonju-si Jeollabuk-do 54871 (KR)
(74) Representative: ETL Wablat & Kollegen Patent- und Rechtsanwaltsgesellschaft mbH
(86) International application number: PCT/KR2017/002563
(87) International publication number: WO 2017/155328

(57) **Abstract**

The present invention relates to a method for preparing a biocompatible porcine cartilage-derived extracellular matrix membrane capable of adjusting an in-vovo degradation rate and a mechanical property, and a composition containing the porcine cartilage-derived extracellular matrix as an active ingredient, for preventing adhesion between tissues and/or organs. Despite its high biocompatibility as a natural material, cartilage tissue extracellular matrix has a short decomposition period and its mechanical property is weak, thereby restricting the application. Accordingly, a method of enhancing the mechanical property through physical or chemical treatment and radiation treatment has been developed. In the present invention, biomaterials of various formulations were produced by treating the porcine cartilage-derived extracellular matrix with physiochemical methods. In addition, although was carried out, a characteristic that the above cartilage-specific function was maintained despite the treatment of the physico-chemical treatment was checked. Furthermore, it may also be used as an adhesion inhibitor with excellent in-vivo stability and anti-adhesion effect by using the porcine cartilage-derived extracellular matrix material.

## Description

### [Technical Field]

The present invention relates to a method for preparing a biocompatible porcine cartilage-derived extracellular matrix membrane capable of adjusting an in-vovo degradation rate and a mechanical property, and a composition containing a porcine cartilage-derived extracellular matrix as an active ingredient, for preventing adhesion between tissues and/or organs.

### [Background Art]

Natural materials, which are derived from natural substances, animals, or human organisms, have very excellent biocompatibility and physiological functions. As a representative example thereof, an extracellular matrix (ECM) may be taken. The extracellular matrix is a biomaterial extracted from complex human and animal tissues, which can control or regulate cell functions or induce tissue regeneration. Accordingly, a supporter made by using a natural material is evaluated as an ideal cell treatment agent or a material for a tissue engineering supporter because it can provide excellent biofunctionality and biodegradability etc., as well as a less inflammatory reaction after transplantation into a living body. In recent years, techniques have been attracting attention in which allogenic or xenogenic tissues or organs are harvested, and cells are then acellularized and used as various types of supporters or membranes.

In the meantime, cartilage-derived extracellular matrix is an anechoic and non-nervous tissue, which is different in tissue specificity from extracellular matrix of other issues. Representatively, cartilage tissue is rich in chondromodulin, thrombospondin, endostatin, and the like, which inhibit angiogenesis, and further includes lubricin, biglycan, decorin, fibromodulin, etc. which prevent cell adhesion. However, despite its high biocompatibility and functionality as a natural material, the extracellular matrix of the cartilage tissue is difficult to adjust a degradation time and its mechanical property is weak, which limits human body application. Accordingly, it is necessary to develop a method of controlling the degradability and improving the mechanical property through physical or chemical treatment thereof.

Adhesion is a condition in which tissues of organs to be separated are connected and fused to fibrous tissues by surgical operation or inflammation. Complications thus generated are small bowel obstruction, acquired female infertility, ectopic pregnancy, chronic abdominal pain, reoperation, or the like. The adhesion occurs 50 to 90 % after surgical operation, and occurs more often as the number of patients who undergo reoperation by adhesion reaches 34.6 %. The adhesion is similar to a healing process that is performed after tissue damage, so it is important to prevent the adhesion without interfering with the healing process. The adhesion occurs when cellulose that is normally deposited during the healing process of a surgical site is continuously developed despite the completion of the healing process, causing it to be combined with adjacent tissues and then ultimately joined to a complete single tissue or organ through penetration of blood vessel. Drugs that accelerate cellulose degradation may be used in order to prevent such adhesion, but in many cases, an anti-adhesion material are used to space tissues by creating physical barriers between the tissues.

As the conventional anti-adhesion material, a biodegradable material such as cellulose or a hyaluronic acid is often used to serve as a physical barrier, or a natural material such as collagen is widely used. Such a biodegradable material has a property of absorbing water and is characterized by hydrolysis thereof, and is used mainly after abdominal surgery because of their rapid degradation rate. These products are usually in the form of a gel, which has a disadvantage in that they are widely applied and difficult to fix to the damaged area, so that the commercialized products are not effective in preventing adhesion. For example, in the case of Interceed, which is the most commonly adhesion inhibitor, the anti-adhesion effect from its use is only 60% (www.ethicon.com).

In the meantime, some tissues, e.g., musculoskeletal tissues, spinal canal, and teeth, have a long regeneration period and require long-term exercise. Since their regenerations occur at different periods of time and these are parts at which movement occurs, the adhesion inhibitor for each tissue must have necessary properties of the tissue

For example, injuries to the musculoskeletal tissues require several weeks or months of regeneration. During this period, adhesion of ligament or tendon are often accompanied. In the case of surgical invasion of joints, rehabilitation exercises over many months are often necessary.

Therefore, a biomaterial that may remain without being decomposed during this period is necessary. Conventional adhesion inhibitors have rapid degradability and may not be suitable for such tissues

The final stage of adhesion is vasculogenesis, and at this stage, an adhered tissue is completely formed into a single tissue, and the adhesion is irreversibly completed. It is therefore very important to prevent formation of blood vessels passing through damaged tissues. However, the conventional adhesion inhibitors do not have a pharmacological mechanism to prevent ultimate infiltration of blood vessels because they act as simple barriers but have no physiological functions.

### [Prior art document]

### [Patent document]

(Patent document 0001) Korean Patent Application Publication No. 10-2010-0041027 (2010.04.22)

### [DISCLOSURE]

### [Technical Problem]

An aspect of the present invention is to provide a method for preparing a biocompatible porcine cartilage-derived extracellular matrix membrane capable of adjusting an in-vovo degradation rate and a mechanical property, and a composition containing a porcine cartilage-derived extracellular matrix as an active ingredient, for preventing adhesion between tissues and/or organs.

### [Technical Solution]

An exemplary embodiment of the present invention provides a method for preparing a biocompatible porcine cartilage-derived extracellular matrix membrane capable of adjusting an in-vovo degradation rate and a mechanical property, including: separating porcine cartilage; lyophilizing and pulverizing the separated porcine cartilage; decellularizing the pulverize porcine cartilage powder; preparing an aqueous solution of porcine cartilage powder by mixing the decellularized porcine cartilage powder with an acidic solution and pepsin and then by neutralizing it with a basic solution; preparing a porcine cartilage-derived extracellular matrix membrane by mixing the aqueous solution of porcine cartilage powder with a crosslinking agent; and irradiating the porcine cartilage-derived extracellular matrix membrane with radiation.

In addition, an exemplary embodiment of the present invention provides a composition containing the porcine cartilage-derived extracellular matrix prepared by the above method as an active ingredient, for preventing adhesion between tissues and/or organs.

### [Advantageous Effects]

The present invention relates to a method for preparing a biocompatible porcine cartilage-derived extracellular matrix membrane capable of adjusting an in-vovo degradation rate and a mechanical property, and a composition containing the porcine cartilage-derived extracellular matrix as an active ingredient, for preventing adhesion between tissues and/or organs. Despite its high biocompatibility and functionality as a natural material, cartilage tissue extracellular matrix has difficulty in controlling a decomposition period and its mechanical property is weak, thereby restricting the application. Accordingly, a method of enhancing the mechanical property through physical or chemical treatment and radiation treatment has been developed. In the present invention, biomaterials of various formulations were produced by treating the porcine cartilage-derived extracellular matrix with physiochemical methods. In addition, although was carried out, a characteristic that the above cartilage-specific function was maintained despite the treatment of the physico-chemical treatment was checked. Furthermore, it may also be used as an adhesion inhibitor with excellent in-vivo stability and anti-adhesion effect by using the porcine cartilage-derived extracellular matrix material.

### [Description of the Drawings]

FIG. 1 illustrates a preparing process of a porcine cartilage-derived extracellular matrix membrane according to an exemplary embodiment of the present invention.
FIG. 2 is a result of comparing before and after crosslinking of the cartilage extracellular matrix membrane. Extracellular matrix anti-adhesion membrane, (Left) Before cross-linking, (Middle) After cross-linking, (Right) After irradiation.
FIG. 3 is a result of analyzing ingredients of a source material of the cartilage extracellular matrix.

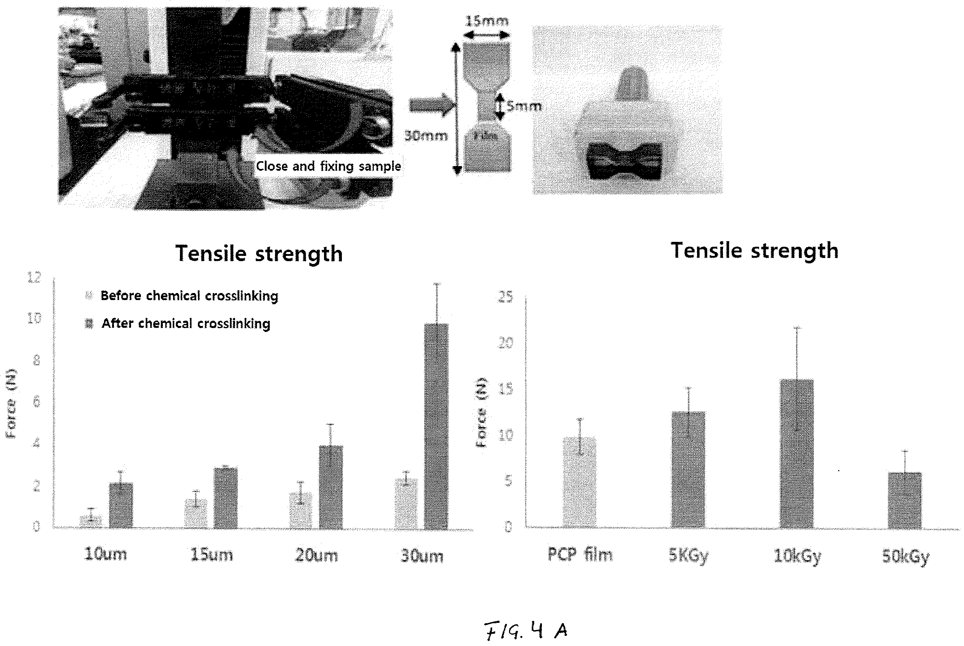

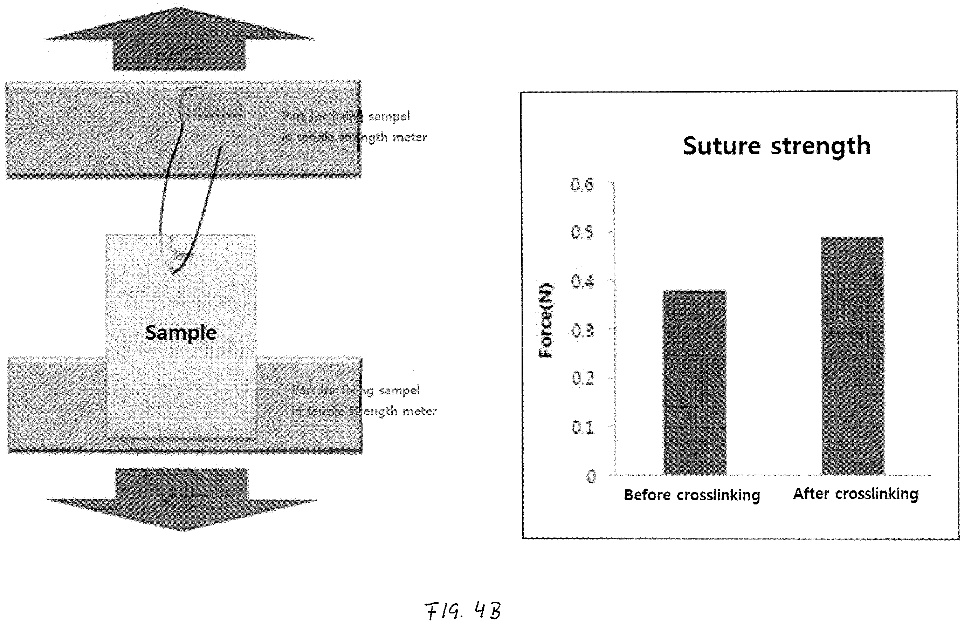

FIG. 4 is a result of mechanical property analysis by before and after crosslinking of the cartilage extracellular matrix membrane. (a) illustrates a result of tensile strength analysis according to before and after cross-linking and a thickness of the cartilage extracellular matrix membrane. [Top] (Left) tensile strength meter, (Middle) Sample cut shape for tensile strength measurement, (R) sample cutter for tensile strength measurement. [Below] tensile strength measurement result after chemical crosslinking. (b) illustrates a result of suture strength measurement according to crosslinking of the cartilage extracellular matrix membrane.
FIG. 5 is a result of an enzymatic degradation test according to irradiation of the cartilage extracellular matrix membrane.
FIG. 6 is a result of an in-vovo degradation test according to irradiation of the cartilage extracellular matrix membrane.
FIG. 7 is a result of a vascular endothelial cell attachment test of the cartilage extracellular matrix membrane.
FIG. 8 is a result of an in-vivo effect checking test using a mouse cecum adhesion model. (a) illustrating a mouse cecum adhesion model test process and an extracellular matrix membrane transplantation process. (b) is a comparative photograph with a control group after one week of the extracellular matrix membrane transplantation. (c) illustrates a comparative photograph of histological analysis with a control group after one week of the extracellular matrix membrane transplantation.

### [Mode for Invention]

An exemplary embodiment of the present invention provides a method for preparing a biocompatible porcine cartilage-derived extracellular matrix membrane capable of adjusting an in-vovo degradation rate and a mechanical property, including: separating porcine cartilage; lyophilizing and pulverizing the separated porcine cartilage; decellularizing the pulverize porcine cartilage powder; preparing an aqueous solution of porcine cartilage powder by mixing the decellularized porcine cartilage powder with an acidic solution and pepsin and then by neutralizing it with a basic solution; preparing a porcine cartilage-derived extracellular matrix membrane by mixing the aqueous solution of porcine cartilage powder with a crosslinking agent; and irradiating the porcine cartilage-derived extracellular matrix membrane with radiation.

It may be preferable that the crosslinking agent is glutaraldehyde, but the prevent invention is not limited thereto.

It may be preferable that the irradiating is performed by using gamma rays of 5 to 100 KGy, but the prevent invention is not limited thereto.

In the meantime, an in-vovo degradation rate and a mechanical property of an in-vivo membrane may be adjusted by treating the porcine cartilage-derived extracellular matrix membrane with radiation, thereby obtaining a sterilization effect.

In an exemplary embodiment of the present invention, the decellularizing is performed by a physical decellularization method, a chemical decellularization method, or a combination of physical and chemical methods.

The physical decellularization method includes freeze-thawing, ultrasonication, or physical stirring. The chemical decellularization method is performed by treating the porcine cartilage-derived powder with a hypotonic buffer, anionic surfactant, non-ionic surfactant, cationic surfactant, DNase, RNase or trypsin. In addition, it may be preferable that the decellularizing is performed at a temperature range of about 0 to 50 °C.

In the chemical decellularization method, the hypotonic buffer may be a Tris HCl (pH 8.0) solution, the anionic surfactant may be sodium dodecyl sulfate (SDS), sodium deoxycholate, or Triton X- 200, the non-ionic surfactant may be Triton X-100, and the cationic surfactant may be selected from the group consisting of CHAPS, Sulfobetaine-10 (SB-10), Sulfobetaine-16 (SB-16), or Tri-n-butyl phosphate.

In addition, an exemplary embodiment of the present invention provides a composition containing a porcine cartilage-derived extracellular matrix as an active ingredient, for preventing adhesion, prepared according to the above method.

Specifically, the porcine cartilage-derived extracellular matrix may prevent adhesion formation by inhibiting fibrosis and inflammation of the surgical site.

It may be preferable that the composition is in any form selected from the group consisting of ointments, powders, gels, films, slabs, wraps and sponges.

Hereinafter, the present invention will be described in detail according to examples which do not limit the present invention. It should be understood that the following examples of the present invention are only for the purpose of illustrating the present invention and do not limit or restrict the scope of the present invention. It is therefore to be understood that what can be easily inferred by those of ordinary skill in the art to which the invention pertains from the following detailed description and examples of the present invention is included within the spirit and scope of the invention as defined by the appended claims.

### <Example 1> Preparing porcine cartilage-derived extracellular matrix membrane

### 1. Separating porcine cartilage

In order to produce porcine cartilage-derived extracellular matrix powder, porcine knee cartilage of a facility conforming the standard was purchased and used referring to "Animal tissues and their derivatives utilized in the manufacture of medical devices, part 1; Analysis and management of risk, part 2; controls on sourcing, collection and handling" of EN 12442.

### 2. Separation and pulverization of porcine cartilage

A preparing process of porcine cartilage powder will be described as follows.

A cartilage fragment (about 20 X 30 mm) was prepared by cutting cartilage from the porcine cartilage, and washed with a physiological saline solution for 10 minutes, frozen at -80 °C, and lyophilized for 3 days. The lyophilized cartilage fragment was frozen and pulverized to a size of about 10 µm using a freezing mill (JAI, JFC-300, JAPAN) and stored at -80 °C.

### 3. Physico-chemical decellularization of porcine cartilage powder

A decellularization process was performed as follows in order to remove cells and genetic materials included in the porcine cartilage powder and obtain pure extracellular matrix components.

The porcine cartilage powder prepared was treated with 500 ml of a hypotonic buffer per 10 g and agitated at 200 rpm at 4 ° C for 4 hours. In order to precipitate and separate cartilage powder, it was treated at 10,000 rpm for 30 minutes using a centrifugal separator (US-21SMT, Vision, Korea).

Supernate was removed, and then the cartilage powder was added to 0.1% SDS (sodium dodecyl sulfate, Bio-Rad, USA) solution and agitated at 200 rpm at 4 ° C for 2 hours. After the SDS treatment, the cartilage powder was washed five times using third distilled water. The exchange of the washing water was performed under the centrifugal conditions as described above.

Next, 200 ml of 500 U / ml of DNase (Sigma, USA) was treated, and it was agitated at 200 rpm in an incubator of 37 °C for 12 hours. After the Dnase treatment, it was washed 5 times with the third distilled water as described above.

Next, the decellularized cartilage powder was cooled in a cryocooler of -80 °C and lyophilized for 3 days. The dried cartilage powder was pulverized in the same manner as described above to finally obtain the cartilage powder having a size of about 10 µm and stored at -80 °C as needed.

### 4. Preparing water-soluble cartilage powder using enzyme

Pepsin (Sigma, USA) was treated with 100 ml of a hydrochloric acid aqueous solution per 4 g of decellularized cartilage powder and agitated at 200 rpm at 4 °C for 24 hours.

After pepsin treatment, it was neutralized to pH 7.4 by using a NaOH solution. The water-soluble cartilage powder was added to the dialysis membrane (MWCO 1000, Spectrolab, USA) and agitated at 200 rpm at 4 °C for 24 hours in the third distilled water. Hereinafter, the water-soluble cartilage powder was placed in a container, cooled in the cryocooler of -80 °C, and lyophilized for 3 days. The water-soluble cartilage powder was pulverized in the same manner as described above to finally obtain the cartilage powder having a size of about 10 µm and stored at -80 °C as needed.

### 5. Preparing porcine cartilage extracellular matrix membrane

1.3 g of the water-soluble cartilage powder prepared above was treated with 100 ml of distilled water, and then agitated at 200 rpm at room temperature for 1 hour.

The aqueous solution of cartilage powder was put in a vessel of the centrifugal separator and treated at 3000 rpm for 10 minutes. Using a pipet, the supernatant was dispensed in a volume of 35 ml into a square silicone mold of 100 x 100 mm and dried in a clean bench for 48 hours.

1 ml of a glutaraldehyde solution (Sigma, USA) of 0.1 % per 6 mg of the dried membrane was treated and agitated at 100 rpm at room temperature for 1 hour. The crosslinked membrane was washed 3 times with 1 ml of PBS solution per 6 mg at 100 rpm for 30 minutes, and then washed three times with the third distilled water. The washed membrane was treated with 1 ml of 4M NaCl solution per 6 mg at 100 rpm for 30 minutes at room temperature. The washed membrane was spread on a teflon film in a clean bench and dried to finally produce a porcine cartilage extracellular matrix membrane having a thickness of about 30 µm

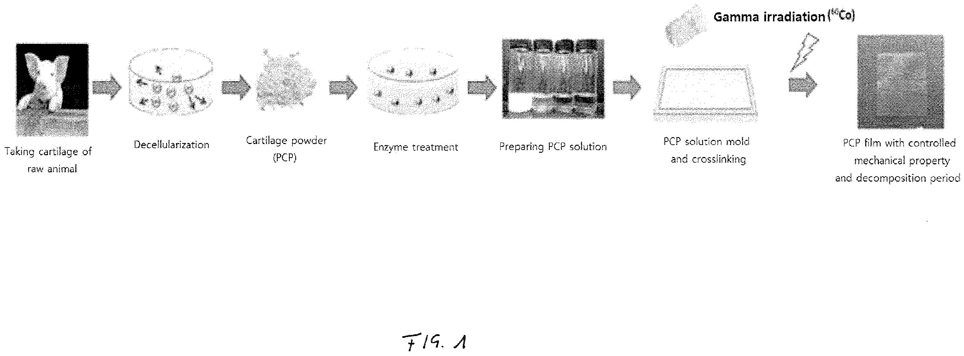

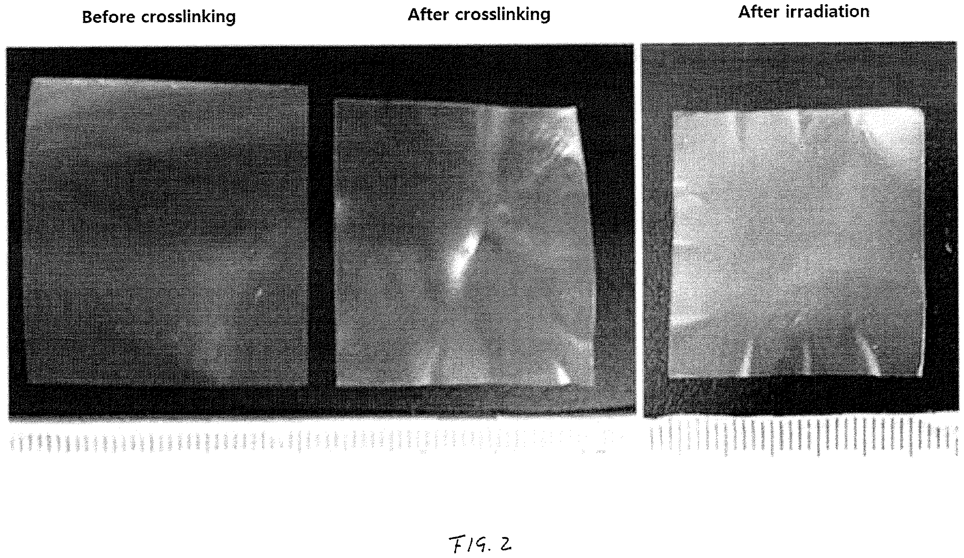

The preparing process of the porcine cartilage extracellular matrix membrane is the same as illustrated in FIG. 1, and results of the comparison before and after crosslinking of the cartilage extracellular matrix membrane are the same as illustrated in FIG. 2.

### 6. Radiation treatment of porcine cartilage extracellular matrix membrane (Adjustment of degradation of membrane and sterilization)

The extracellular matrix membrane prepared above was packed with silver foil and irradiated with gamma rays at a dose of 5 KGy to 100 KGy.

### <Example 2> Analysis of ingredients of source material of cartilage extracellular matrix

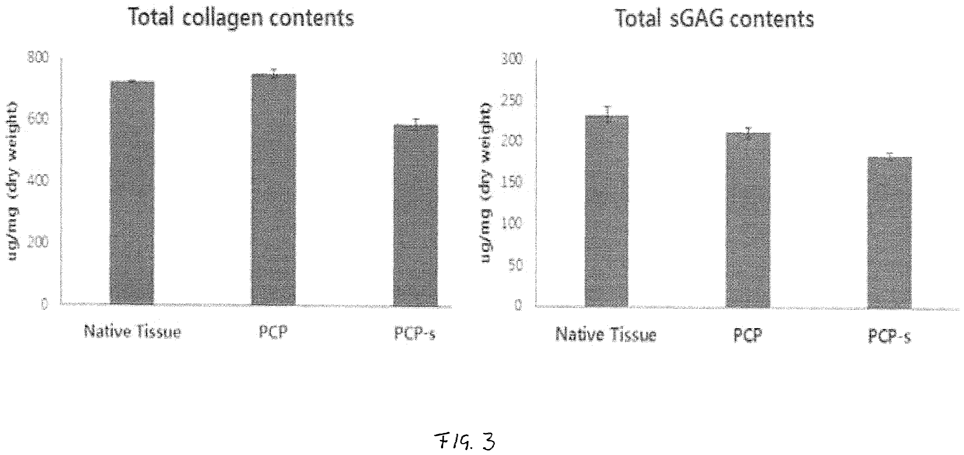

As a result of ingredient analysis of source materials according to the process of cartilage extracellular matrix, it was seen that collagen and glycoprotein components, which occupied a largest portion of the cartilage extracellular matrix, were maintained without loss. The collagen was measured by sirius red assay by dissolving the source material in an acid solution and pepsin enzyme, and the glycoprotein was dissolved in papain solution and measured by DMMB assay (FIG. 3).

### <Example 3> Analysis of mechanical properties according to before and after crosslinking of cartilage extracellular matrix membrane

### 1. Analysis of tensile strength before and after crosslinking and thickness of cartilage extracellular matrix membrane

Since the membrane used as an adhesion inhibitor is important in terms of the physical properties that can sufficiently protect the surgical site, a tensile strength test was performed on the basis of the guidelines of Ministry of Food and Drug Safety (FIG. 4A).

As illustrated in FIG. 4A, the cut extracellular matrix membrane was plotted by using a tensile strength meter to show the degree of tensile strength as an ultimate force value. As shown in the results, it is seen that the tensile strength is improved as the thickness of the extracellular matrix increases, and the physical strength is also significantly increased by chemical crosslinking. In addition, it is seen that the physical strength is controllable according to the radiation dose in the measurement of the tensile strength of the sample subjected to the irradiation treatment (FIG. 4A).

### 2. Measurement of suture strength by crosslinking of cartilage extracellular matrix membrane

Since the adhesion-preventing membrane should be fixed to the treatment site, it is effective to prevent the adhesion, so that the membrane is strong enough to be maintained without being broken even when the suture is performed at the time of surgery. Since the suture strength was not specified separately at the Ministry of Food and Drug Safety, a protocol that can measure the suture strength by itself has been established and measured

The suture strength was measured by cutting the film as follows, sealing the film with a surgical thread, and then measuring the tensile strength by attaching the surgical thread and the extracellular matrix membrane to the tensile strength meter, respectively

As a result of the measurement, it is seen that the suture strength of the extracellular matrix membrane after crosslinking is significantly higher than that before crosslinking (FIG. 4B).

### <Example 4> Enzymatic degradation test according to irradiation of cartilage extracellular matrix membrane

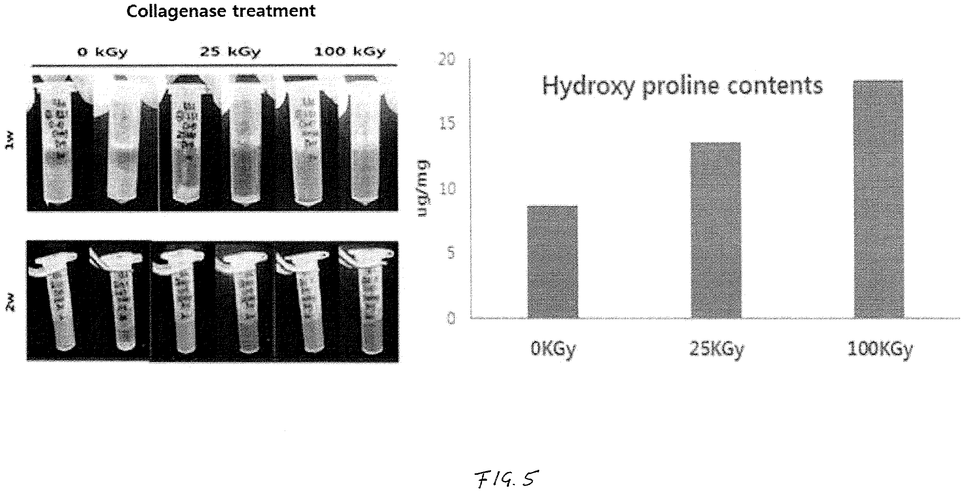

Since the degradation time varies depending on a site to be treated, an adhesion inhibitor having a controlled decomposition period for each organ should be used in order to achieve a specific long-term inhibition effect. Collagenase was treated in vitro and the decomposition behavior according to time was examined in order to examine whether the degree of degradation can be adjusted according to the irradiation doses of the cartilage extracellular matrix membrane. Tests were performed by cutting the cartilage extracellular matrix membrane treated with different doses of radiation by 1X1 cm and then treating it to collagenase-treated PBS to observe it for 2 weeks (Left in FIG. 5). In addition, the sample treated with collagenase for 2 weeks was centrifuged to analyze the amount of hydroxyproline in the supernatant, such that the degree of degradation of collagen, which is a main ingredient of the extracellular matrix membrane, was measured (Right in FIG. 5). As a result of the measurement, it is seen that the degree of degradation of the extracellular matrix membrane was controlled in the treatment of collagenase according to the irradiation dose.

### <Example 5> In-vivo degradation test according to irradiation

Tests were performed as follows in order to check a difference of in-vivo biodegradation according to irradiation of extracellular matrix membrane.

logas sterilization was performed on the cartilage extracellular matrix membrane prepared in FIG. 1 and the membrane obtained by irradiating the cartilage extracellular matrix. After subcutaneous injection of Rat, each membrane was transplanted thereto, and then sutured again to observe biodegradation for 4 weeks

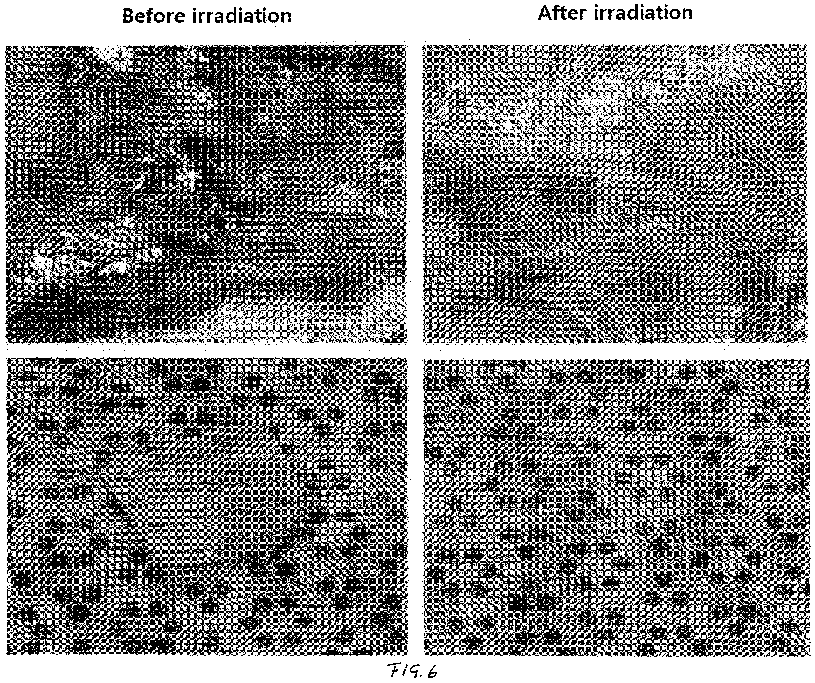

As a result, it is seen that the degree of biodegradation in the in-vivo subcutaneous tissue was controlled according to the irradiation (FIG. 6).

### <Example 6> Vascular endothelial cell adhesion experiment on extracellular matrix membrane

A difference of vascular endothelial cell adhesion acting in an adhesion mechanism using the cartilage extracellular matrix membrane prepared in Fig. 1 was checked as follows.

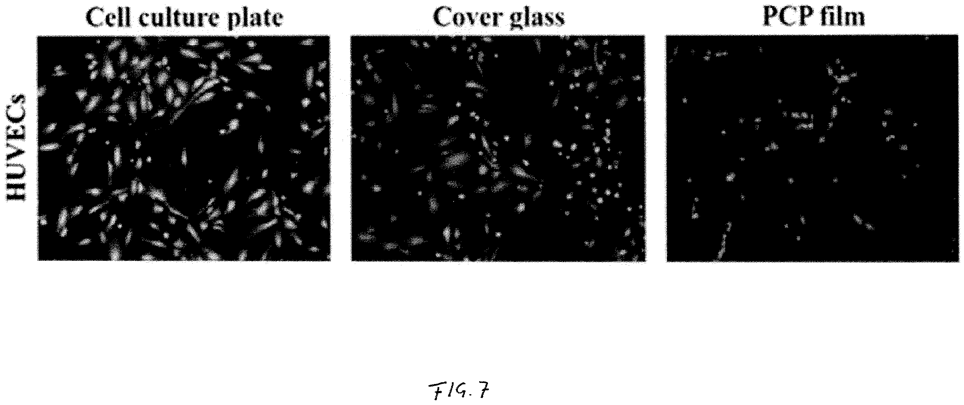

Cover glass and the cartilage extracellular matrix membrane prepared in FIG. 1 were attached to a separate 24-well dish having a diameter of 5 mm, dried and fixed, and sterilized by iogas. 2x104 vascular endothelial cells were transplanted onto a film-coated dish, the cover glass, and a non-coated 24-well plate and adhered for 24 hours. The cells attached to each surface were then stained with calein and observed with a fluorescence microscope. As a result, it was seen that cell attachment was inhibited in the dish coated with the extracellular matrix membrane compared to the cover glass and the well plate (FIG. 7).

### <Example 7> Test for checking in-vivo effect using mouse cecum adhesion model

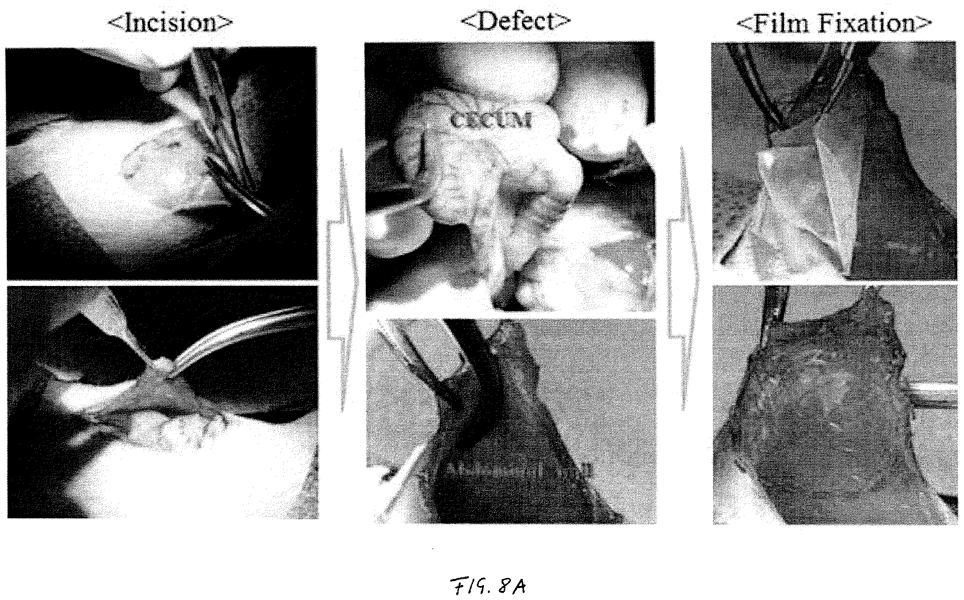

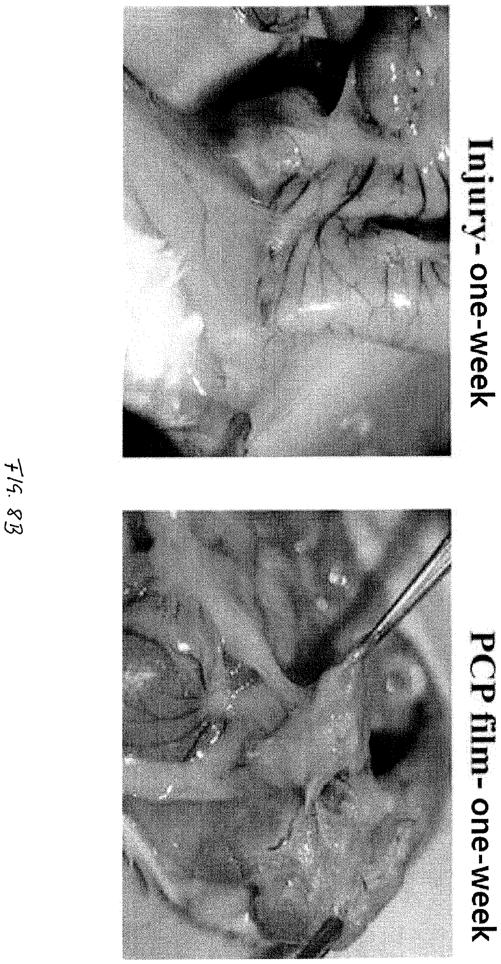

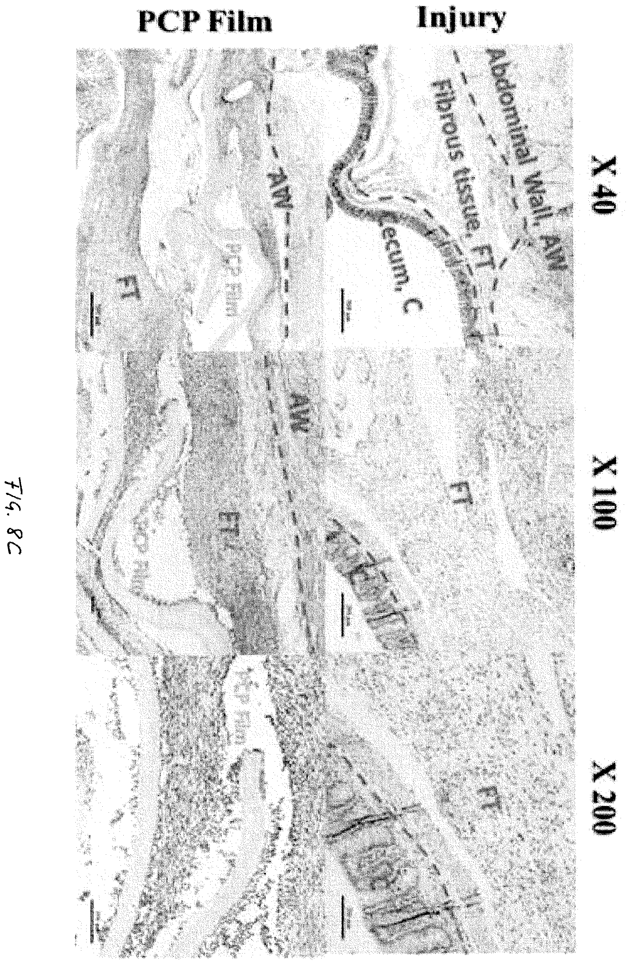

(1) An adhesion model was prepared using a C57BL6 mouse of 8 weeks. After a skin layer and a muscle layer of the abdomen of the mouse were respectively incised and then were sutured to create a model in which adhesion occurs between the damaged tissues. Then, an adhesion effect was checked. Adhesive tissues developed for one week, and the adhesion model in which muscle and/or skin were strongly attached were prepared (FIG. 8A).
(2) In the subcutaneous adhesion model prepared above, one week after transplantation of the cartilage extracellular matrix membrane to the site where the adhesive tissue between the muscle layer and the skin layer was formed, a one-week result was checked. In the group inducing only adhesion, adhesive tissue wrapped the wound and was thickly generated for 1 week. When the cartilage extracellular matrix membrane is transplanted, it can be seen that the muscle layer and the skin layer are separated and no adhesion tissue is formed (FIG. 8B).
(3) Cells and cytoplasm of the tissue obtained in the above were stained with hematoxylin and eosin. As a result, the cells were concentrated on the cartilage extracellular matrix membrane itself to physically defend the muscle layer and the skin layer, thereby preventing the formation of adhesive tissue between two tissues (FIG. 8C).

## Claims

1. A method for preparing a biocompatible porcine cartilage-derived extracellular matrix membrane capable of adjusting an in-vovo degradation rate and a mechanical property, the preparing method comprising:
separating porcine cartilage;
lyophilizing and pulverizing the separated porcine cartilage;
decellularizing the pulverize porcine cartilage powder;
preparing an aqueous solution of porcine cartilage powder by mixing the decellularized porcine cartilage powder with an acidic solution and pepsin and then by neutralizing it with a basic solution;
preparing a porcine cartilage-derived extracellular matrix membrane by mixing the aqueous solution of porcine cartilage powder with a crosslinking agent; and
irradiating the porcine cartilage-derived extracellular matrix membrane with radiation.

2. The preparing method of claim 1, wherein the crosslinking agent is glutaraldehyde.

3. The preparing method of claim 1, wherein the irradiating is performed by using gamma rays of 5 to 100 KGy.

4. A composition containing the porcine cartilage-derived extracellular matrix of any one of claim 1 to claim 3 as an active ingredient, for preventing adhesion between tissues and/or organs.

5. The composition of claim 4, wherein the composition is in any form selected from the group consisting of ointments, powders, gels, films, slabs, wraps and sponges.
